# FASCICULE DE BREVET EUROPEEN

(11) **EP 3 071 676 B1**
(45) Date de publication et mention de la délivrance du brevet: **05.09.2018**
(21) Numéro de dépôt: 14809471.7
(22) Date de dépôt: 06.11.2014
(51) Int. Cl.: C10L 3/08, C10L 3/10, B01D 53/22, C12P 5/02

(54) **PROCÉDÉ DE PRODUCTION DE BIOMÉTHANE INTÉGRANT LA PRODUCTION DE CHALEUR POUR LE MÉTHANISEUR UTILISANT UNE SÉPARATION PAR MEMBRANE**
VERFAHREN ZUR HERSTELLUNG VON BIOMETHAN MIT ERZEUGUNG VON WÄRME FÜR DEN METHANISIERER MITTELS MEMBRANTRENNUNG
METHOD FOR PRODUCING BIOMETHANE INCORPORATING THE PRODUCTION OF HEAT FOR THE METHANISER USING MEMBRANE SEPARATION

(30) Priorité: 18.11.2013 FR 1361271
(43) Date de publication de la demande: 28.09.2016
(73) Titulaire: L'Air Liquide Société Anonyme pour l'Etude et l'Exploitation des Procédés Georges Claude, 75007 Paris (FR)
(72) Inventeur: PAGET, Nicolas, 38400 Saint Martin D'Hères (FR); GARNAUD, Delphine, F-38000 Grenoble (FR); PRINCE, Guénaël, F-38120 Saint Egreve (FR); LEFEBVRE, Mathieu, F-38330 Saint Nazaire Les Eymes (FR)
(74) Mandataire: Laigneau, Amandine
(86) Numéro de dépôt international: PCT/FR2014/052829
(87) Numéro de publication internationale: WO 2015/071575

(56) Documents cités:
- EP-A1- 1 634 946
- DE-A1-102007 058 548
- DE-U1-202008 016 134
- JP-A- 2009 242 773
- A. MOLINO ET AL: "Biomethane production by anaerobic digestion of organic waste", FUEL, vol. 103, 1 janvier 2013 (2013-01-01), pages 1003-1009, XP055127374, ISSN: 0016-2361, DOI: 10.1016/j.fuel.2012.07.070

## Description

La présente invention concerne un procédé de production de biométhane apte à alimenter un réseau de gaz naturel intégrant un procédé de fourniture de chaleur pour le chauffage de l'étape de production de biogaz; le procédé comprend au moins des étapes de production de biogaz par fermentation anaérobique de matière organique, de prétraitement et compression du biogaz ainsi que de perméation pour obtenir après une première séparation un flux de biométhane et un perméat gazeux à teneur en méthane réduite; dans le même temps, le procédé fournit la chaleur nécessaire à l'étape de fermentation anaérobique.

Le biomethane est un gaz riche en méthane obtenu via une épuration adaptée à partir de biogaz, il a les mêmes caractéristiques que le gaz naturel auquel il peut être substitué.

Aux valorisations - essentiellement sur site ou à proximité - du biogaz, s'est ainsi ajoutée plus récemment celle de ce biogaz épuré aux spécifications du gaz naturel. Le biométhane produit peut ainsi être utilisé en tant que substitut non fossile du gaz naturel complétant les ressources de gaz naturel avec une partie renouvelable produite au coeur des territoires. Il est utilisable pour exactement les mêmes usages.

En particulier, dans le cadre de sa valorisation, le biométhane - substitut renouvelable au gaz naturel, ayant les mêmes caractéristiques que celui-ci - peut être injecté dans un réseau de distribution ou de transport de gaz naturel qui permet de relier producteurs de gaz et consommateurs.

Un réseau de distribution ou de transport de gaz naturel permet d'approvisionner des consommateurs en gaz naturel. Le réseau est maintenu à une pression comprise entre 2 et 6 bars pour la distribution, 15 et 25 bars pour la distribution moyenne pression et 25 à 80 bars pour le transport.

Le biogaz est quant à lui un gaz produit par la fermentation naturelle ou artificielle de matières organiques végétales ou animales (la méthanisation). Le composant caractéristique des biogaz est le méthane qui est formé lors de la dégradation biochimique des déchets organiques, l'autre constituant principal est le dioxyde de carbone. Un biogaz contient également, mais en moindre proportion, de l'eau, de l'azote, de l'hydrogène sulfuré, de l'oxygène, ainsi que des composés organiques autres, à l'état de traces.

Selon les matières organiques et les techniques utilisées, les proportions des composants diffèrent, mais en moyenne un biogaz comporte, sur gaz sec, de 30 à 75% de méthane, de 15 à 60% de CO₂, de 0 à 5% d'azote, de 0 à 5% d'oxygène et des composés traces.

Le biogaz est produit par méthanisation de matière organique, c'est à dire par fermentation anaérobique. Elle est réalisée dans une cuve fermée appelée aussi méthaniseur ou digesteur. Il est nécessaire de procéder en l'absence d'air (procédé anaérobie) et de maintenir une température stable dans l'enceinte du réacteur. Cette température dépend du procédé de méthanisation, c'est-à-dire du type de bactéries utilisées pour la dégradation de la matière organique, mais dans tous les cas il sera nécessaire d'apporter de la chaleur; c'est ainsi que pour un procédé mésophile la température doit être maintenue entre 30 et 37°C, tandis qu'un procédé thermophile requiert une température de 50 à 55°C.

Ainsi donc, l'ajout permanent de matière organique à une température inférieure à celle du méthaniseur ainsi que la température extérieure contribuant au refroidissement dudit méthaniseur, il est nécessaire de prévoir de fournir de la chaleur pour l'étape de production de biogaz. La chaleur à fournir peut ainsi représenter de 5 à 20 % de l'énergie contenue dans le biogaz produit.

Parallèlement, afin de pouvoir bénéficier de tarifs subventionnés pour l'achat de biométhane lors de l'injection au réseau, il est nécessaire que la source fournissant la chaleur pour la méthanisation utilise des énergies renouvelables.

Pour répondre à cette double problématique, la source de chaleur la plus communément utilisée est le biogaz produit par le digesteur. Dans ce cas, la chaleur est fournie au procédé de méthanisation par échange de chaleur avec de l'eau de chaudière, celle-ci étant chauffée en utilisant du biogaz prélevé avant épuration sur le biogaz produit. La part de biogaz ainsi prélevée avant épuration ne participe donc pas à la production finale.

Or, pour pouvoir valoriser valablement sa production sous la forme de gaz naturel de substitution, un producteur de biogaz doit pouvoir disposer d'une production la plus importante possible parce que (i) l'épuration de biogaz coûte cher en terme d'investissement et (ii) il faut être en mesure de fournir des quantités suffisantes de biométhane pour disposer de débouchés - tout particulièrement dans le cas de petites productions.

Pouvoir utiliser la plus grande part possible de biogaz pour produire du biométhane est donc essentiel, et même impératif pour un petit producteur dont la production de biogaz peut être de l'ordre de vingt Nm3/h à quelques dizaines de Nm3/h. Pour ces petits producteurs, pouvoir utiliser la plus grande partie possible du méthane produit, jusqu'à 99% du méthane produit ou même plus peut être un atout majeur.

Dans le même temps, pour bénéficier de tarifs subventionnés pour l'achat de biométhane lors de l'injection au réseau, il reste impératif de faire appel à une source de chaleur utilisant des énergies renouvelables.

Des solutions existent qui proposent de récupérer de la chaleur disponible produite par l'unité d'épuration (chaleur du compresseur ou des groupes froids) mais l'apport de chaleur à partir de cette source n'est pas suffisant pour les besoins totaux du digesteur (il ne couvre que 20% environ des besoins).

Une autre solution connue consiste à récupérer la chaleur produite par un système de destruction des évents via une chaudière à bas PCS brûlant un biogaz contenant entre 10 et 20 % de méthane, de préférence 15% ou par oxydation thermique applicable pour des gaz contenant entre 2 et 8 % de méthane, de préférence 5%; cependant, ces solutions de destruction d'évent sont coûteuses, trop coûteuses pour les petits projets . En outre, elles ne permettent de fournir qu'une petite partie de la chaleur nécessaire au méthaniseur (la solution couvre au maximum 15% des besoins).

Le document EP_A_1 634 946 divulgue un procédé de production de biométhane ayant qualité de gaz naturel à partir d'un biogaz issu d'un réacteur de fermentation de biomasse. Au sein de ce procédé, il s'agit de produire un gaz pauvre ayant une concentration en méthane allant de 10 à 15% volumétrique. Une teneur en méthane aussi basse requiert l'utilisation des brûleurs spéciaux (brûleur de gaz pauvre sans flamme).

Il n'existe donc pas à ce jour de solution permettant de fournir la totalité de la chaleur nécessaire au méthaniseur qui à la fois:
- est issue du procédé de production du biométhane et utilise de ce fait les énergies renouvelables
- garde disponible la totalité de la production de biogaz pour l'épuration,
- n'implique pas de surcoûts importants, qui seraient excessifs pour un petit producteur, et donc ne fait pas appel à des traitements complémentaires coûteux pour fournir le moyen de chauffage du méthaniseur.

Un but de l'invention est de pallier ces insuffisances et de proposer au producteur de biométhane une solution lui permettant de chauffer la charge présente dans le méthaniseur qui réponde aux besoins énoncés.

Selon l'invention, il est pour cela proposé un procédé de production de biométhane destiné à alimenter un réseau de gaz naturel et intégrant un procédé de fourniture de chaleur pour le chauffage de l'étape de production de biogaz dans lequel :
- le procédé de production de biométhane comprend au moins:
- une étape (a) de production de biogaz par méthanisation de matière organique,
- une étape (b) de prétraitement de la totalité du biogaz produit lors de l'étape (a),
- une étape (c) de compression de la totalité du biogaz prétraité,
- une étape (d) de séparation par perméation de la totalité du biogaz prétraité et comprimé issu de l'étape (c) pour produire un rétentat gazeux à teneur en méthane supérieure à 89%, de préférence supérieure à 96,5% et un perméat gazeux à teneur en méthane comprise entre 10 et 25%, de préférence de l'ordre de 20%,
- une étape (e) de mise à disposition du rétentat gazeux de l'étape (d) en tant que biométhane,
- le procédé de chauffage de l'étape (a) de production de biogaz comprend au moins :
- une étape (f) d'apport de chaleur à l'étape (a) via un circuit d'eau de chauffage,
- une étape (g) de chauffage de ladite eau de chauffage dans une chaudière à gaz, caractérisé en ce que ledit procédé de fourniture de chaleur comprend aussi au moins:
- une étape (h) de séparation par perméation du perméat gazeux issu de la première étape (d) de séparation par perméation pour produire un rétentat gazeux enrichi en méthane dont la teneur en méthane est supérieure ou égale à 25%, de préférence entre 30 et 40%, de préférence encore de l'ordre de 35% et un perméat appauvri en méthane,
- une étape (i) d'alimentation des brûleurs de la chaudière de l'étape (g) avec le rétentat de l'étape de séparation (i),
- une étape (j) d'évacuation du perméat produit par l'étape (h) de séparation par perméation.

Le procédé selon l'invention propose ainsi d'intégrer l'épuration du biogaz en biométhane et la production de chaleur pour le digesteur. La totalité du biogaz est envoyée vers l'épurateur sans détournement d'une partie de la production pour produire de la chaleur ; cela peut constituer une différence importante notamment pour les petits producteurs car lorsque la production est trop faible, elle peut ne pas trouver de débouché vers un réseau.

La technologie d'épuration par membrane permet une séparation efficace du CO₂ et du CH₄. Il est donc possible d'obtenir un biométhane à la qualité requise par l'opérateur de réseau au moyen d'un seul étage de membrane ; en effet, en sortie de ce premier étage de membrane, on produit un gaz enrichi dont la concentration en méthane est supérieure à 89%, pouvant être, en fonction de la qualité requise par le client supérieure à 96,5%.

La concentration en CH₄ dans le perméat de cette première membrane est comprise entre 10 et 25%, de préférence de l'ordre de 20%, et ne permet pas l'utilisation de chaudière à gaz de technologie simple ; celles-ci requièrent en effet une teneur en méthane supérieure à 25% de préférence supérieure ou égale à 30%; afin d'atteindre cette teneur minimale, le procédé de l'invention prévoit d'ajouter sur le perméat un second étage de membranes. Cette solution représente pour le producteur de biogaz un investissement initial et un coût de fonctionnement inférieurs de 30 à 50 % par rapport à un système de purification et un système de chauffage traditionnels. Le rétentat produit par ce deuxième étage de membranes est un gaz enrichi en CH₄ jusqu'à une teneur en méthane supérieure à 25%, de préférence comprise entre 30 et 40 % qui peut être utilisé dans une chaudière à gaz simple. Le perméat de ce second étage est très appauvri en CH₄ il peut être renvoyé à l'atmosphère sans traitement couteux des évents. Moins de 2 %, de préférence moins de 1% du CH₄ produit par le méthaniseur est ainsi renvoyé à l'atmosphère.

Selon les cas, le procédé de l'invention peut comprendre tout ou partie des caractéristiques ci-après.
- Le procédé de chauffage de l'étape (a) comprend une étape (k) d'apport complémentaire de chaleur à l'eau circulant dans ledit circuit d'eau de chauffage à partir de chaleur récupérée lors des étapes (b) et (c). En effet, de la chaleur est dans tous les cas disponible au niveau des équipements tels que compresseur et groupes froids ; elle peut-être utilisée sans investissements excessifs, participant ainsi à la fourniture totale de chaleur au méthaniseur.
- Une fraction variable du retentat enrichi en méthane de l'étape (h) de séparation peut être renvoyée à l'entrée de l'étape de compression (c) lorsque l'offre de chaleur est supérieure aux besoins de l'étape (a) de production de biogaz. En effet, dès lors que les besoins en chaleur du méthaniseur sont pourvus, le supplément de méthane disponible dans le retentat du second étage de membranes est ainsi récupéré pour être recyclé en amont de la séparation membranaire de sorte à accroitre la production de biométhane. A cet effet, une ligne de recirculation équipée d'une vanne à débit variable est pour cela connectée à la canalisation transportant le retentat vers les brûleurs de la chaudière.

Selon un second aspect de l'invention, celle-ci concerne une installation apte à mettre en oeuvre le procédé de l'invention.

En particulier, elle concerne une installation de production de biométhane destiné à alimenter un réseau de gaz naturel et la fourniture intégrée de chaleur pour le chauffage de l'étape de production de biogaz comprenant au moins :
- une unité de production de biométhane comprenant au moins:
- une source de matière organique,
- un méthaniseur pour la production de biogaz par méthanisation de ladite matière organique,
- un module de prétraitement du biogaz produit,
- un compresseur apte à comprimer le biogaz prépurifié,
- un premier module de séparation par perméation membranaire du biogaz prétraité et comprimé apte à produire un rétentat gazeux à teneur en méthane supérieure à 89%, de préférence supérieure à 96,5% et un perméat gazeux à teneur en méthane comprise entre 10 et 25%, de préférence de l'ordre de 20%,
- un moyen de mise à disposition du rétentat gazeux issu dudit premier module de séparation en tant que biométhane produit,
- des moyens aptes à coopérer pour chauffer la matière organique contenue dans le méthaniseur comprenant au moins :
- un circuit d'eau de chauffage apte à chauffer le méthaniseur,
- une chaudière à gaz apte à chauffer l'eau dudit circuit d'eau de chauffage,
caractérisé en ce que les moyens aptes à coopérer pour chauffer ladite matière organique comprennent
- un second module de séparation par perméation membranaire du perméat gazeux issu du premier module de séparation par perméation apte à produire un rétentat gazeux enrichi en méthane dont la teneur en méthane est supérieure ou égale à 25%, de préférence comprise entre 30% et 40%, de préférence encore de l'ordre de 35% et un perméat appauvri en méthane,
- un moyen d'alimentation des brûleurs de ladite chaudière à gaz avec le rétentat du second module de séparation,
un moyen d'évacuation du perméat issu du second module de séparation.

Selon un mode de réalisation avantageux de l'invention, les moyens aptes à coopérer pour chauffer la matière organique contenue dans le digesteur comprennent des moyens complémentaires aptes à récupérer de la chaleur sur le module de prétraitement du biogaz et le compresseur de biogaz prétraité ainsi que des moyens complémentaires aptes à fournir la chaleur récupérée à l'eau du circuit de chauffage du digesteur.

Avantageusement, l'installation comprend en outre des moyens aptes à recycler une fraction du rétentat du second étage de membranes à l'entrée du compresseur de biogaz prétraité, les dits moyens comprenant une canalisation équipée d'une vanne de réglage de débit qui permet, en fonction du besoin de chaleur, de recirculer l'exces de gaz allant vers la chaudière vers l'amont du compresseur. L'objectif est de fournir uniquement la chaleur nécessaire au digesteur.

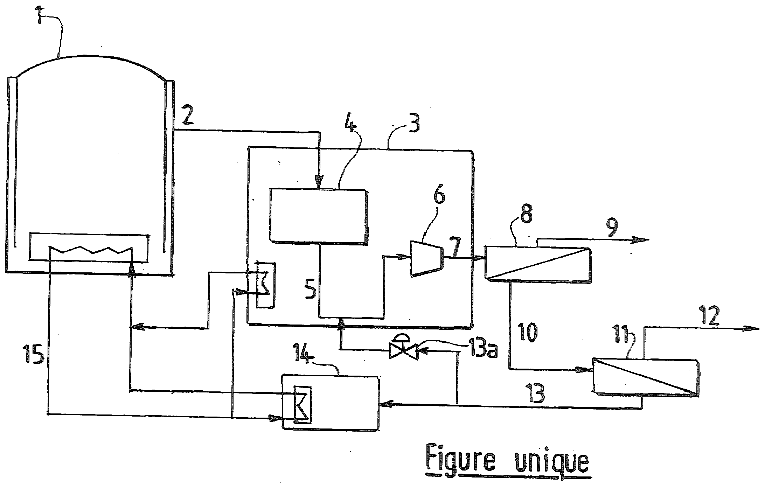

L'invention va maintenant être mieux comprise grâce à la description suivante faite en références à la figure unique jointe.

La figure présente un schéma de principe illustrant différents éléments de l'invention. Dans un but de simplicité ne sont référencés que les éléments de l'installation utiles à la compréhension et à la mise en oeuvre de l'invention.

Selon le schéma de la figure, l'installation de production de biométhane selon l'invention fonctionne de la manière suivante. Le méthaniseur 1 délivre un biogaz 2. Le biogaz 2 est envoyé vers un module 3 de prétraitement dans lequel il subit en 4 différents traitements préalables à une séparation CO₂/CH₄. Le biogaz prétraité 5 est comprimé dans le compresseur 6 pour fournir le biogaz prétraité comprimé 7 à la pression d'entrée dans un premier étage de membrane 8 assurant la séparation CO₂/CH₄. Plus précisément, l'étage de membrane 8 délivre un rétentat gazeux 9 enrichi en méthane dont la teneur en méthane est suffisante pour être substitué au gaz naturel - au moins 89%, si nécessaire 96,5 % ou plus, suivant les spécifications du gaz naturel devant être substitué - et délivre un perméat gazeux 10 à teneur en méthane comprise entre 10 et 25%. Afin de pouvoir utiliser le méthane contenu dans le perméat 10 en tant que combustible dans une chaudière simple, le perméat 10 doit d'abord être enrichi en méthane, il est pour cela envoyé à l'alimentation d'un second étage de membrane 11 qui délivre un perméat 12 appauvri en méthane (moins de 2 % du méthane contenu dans le biogaz produit est ainsi évacué dans le perméat) et un rétentat 13 dont la teneur en méthane est supérieure à 30%, de préférence de l'ordre de 35%. Le flux gazeux 13 - dont la teneur en méthane est ainsi suffisamment élevée- est envoyé aux brûleurs d'une chaudière 14, laquelle peut donc être une chaudière de technologie classique. Le perméat 12 est appauvri en méthane par rapport au flux 11, sa teneur en CH₄ est suffisamment faible pour pouvoir être envoyé à l'atmosphère sans traitement supplémentaire couteux de l'évent. La chaudière 14 fournit de la chaleur au circuit d'eau 15 lequel chauffe la matière organique contenue dans le méthaniseur 1 afin d'y maintenir les conditions de température requises pour le bon déroulement du processus de fermentation anaérobie générant le biogas 2. Un complément de chaleur est fourni pour chauffer l'eau circulant dans le circuit de chauffage 15 à partir de chaleur disponible du module de prétraitement 4 par récupération de chaleur sur des groupes froids et/ou de chaleur de compression sur le module de compression 7.

Lorsque les besoins en chaleur de la fermentation sont inférieurs aux ressources disponibles, une partie 13a du flux 13 est prélevée sur le flux alimentant la chaudière et est envoyée au compresseur 6 pour être épurée en complément du flux de biogaz 5. Ce débit 13a est contrôlé grâce à une vanne de contrôle de débit régulé (vanne FCV1 pour « flow control vanne 1 » en langue anglaise) installée sur la canalisation assurant son recyclage vers le compresseur de sorte à adapter le débit en fonction des besoins de chaleur du méthaniseur. Le tableau ci-dessous présente les caractéristiques des différents flux résultants de la mise en oeuvre de l'invention appliquée à un flux de biogaz prétraité 5 contenant 55% de methane et 44,4% de dioxyde de carbone. Les pourcentages exprimés sont molaires, les teneurs en éléments mineurs ne sont pas indiquées - soit par exemple 0,6% pour le biogaz 5.

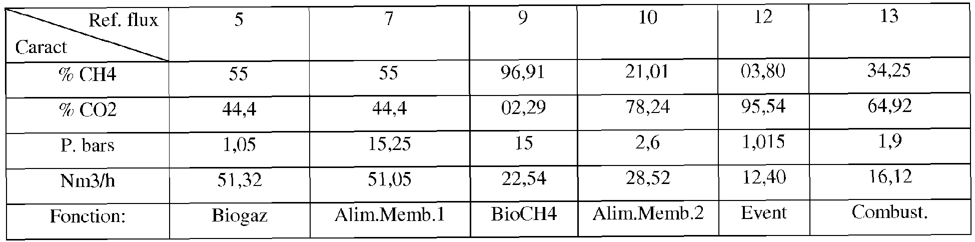

## Revendications

1. Procédé de production de biométhane apte à alimenter un réseau de gaz naturel intégrant un procédé de fourniture de chaleur pour le chauffage de l'étape de production de biogaz dans lequel :
• le procédé de production de biométhane comprend au moins:
• une étape (a) de production de biogaz par méthanisation de matière organique,
• une étape (b) de prétraitement du biogaz produit lors de l'étape (a),
• une étape (c) de compression du biogaz prétraité,
• une étape (d) de séparation par perméation du biogaz prétraité et comprimé issu de l'étape (c) pour produire un rétentat gazeux à teneur en méthane supérieure à 89%, de préférence supérieure à 96,5% et un perméat gazeux à teneur en méthane comprise entre 10 et 25%, de préférence de l'ordre de 20%,
• une étape (e) de mise à disposition du rétentat gazeux de l'étape (d) en tant que biométhane,
• le procédé de fourniture de chaleur à l'étape (a) de production de biogaz comprend au moins :
• une étape (f) d'apport de chaleur à l'étape(a) via un circuit d'eau de chauffage,
• une étape (g) de chauffage de ladite eau de chauffage dans une chaudière à gaz,
**caractérisé en ce que** le procédé de fourniture de chaleur comprend :
• une étape (h) de séparation par perméation du perméat gazeux issu de la première étape (d) de séparation par perméation pour produire un rétentat gazeux enrichi en méthane dont la teneur en méthane est supérieure ou égale à 25%, de préférence comprise entre 30% et 40%, de préférence encore de l'ordre de 35% et un perméat appauvri en méthane,
• une étape (i) d'alimentation des brûleurs de la chaudière de l'étape (g) avec le rétentat de l'étape de séparation (h),
• une étape (j) d'évacuation du perméat produit par l'étape (h) de séparation par perméation.

2. Procédé selon la revendication 1 dans lequel le procédé de chauffage de l'étape (a) comprend une étape (k) d'apport complémentaire de chaleur à l'eau circulant dans ledit circuit d'eau de chauffage à partir de chaleur récupérée lors des étapes (b) et (c).

3. Procédé selon la revendication 1 ou la revendication 2 dans lequel une fraction variable du rétentat enrichi en méthane de l'étape (h) de séparation est renvoyée à l'entrée de l'étape de compression (c) lorsque l'offre de chaleur est supérieure aux besoins de l'étape (a) de production de biogaz.

4. Installation pour mettre en oeuvre le procédé de la revendication 1 de production de biométhane destiné à alimenter un réseau de gaz naturel et la fourniture intégrée de chaleur pour le chauffage de l'étape de production de biogaz comprenant au moins :
• une unité de production de biométhane comprenant au moins:
• une source de matière organique,
• un digesteur pour la production de biogaz par méthanisation de ladite matière organique,
• un module de prépurification du biogaz produit,
• un compresseur apte à comprimer le biogaz prépurifié,
• un premier module de séparation par perméation membranaire du biogaz prépurifié et comprimé apte à produire un rétentat gazeux à teneur en méthane supérieure à 89%, de préférence supérieure à 96,5% et un perméat gazeux à teneur en méthane comprise entre 10 et 25%, de préférence de l'ordre de 20%,
• un moyen de mise à disposition du rétentat gazeux de l'étape (d) en tant que biométhane,
• des moyens aptes à coopérer pour chauffer la matière organique contenue dans le digesteur comprenant au moins :
• un circuit d'eau de chauffage apte à chauffer le digesteur,
• une chaudière à gaz apte à chauffer l'eau dudit circuit d'eau de chauffage,
**caractérisé en ce que** les moyens aptes à coopérer pour chauffer ladite matière organique comprennent :
• un second module de séparation par perméation membranaire du perméat gazeux issu du premier module de séparation par perméation apte à produire un rétentat gazeux enrichi en méthane dont la teneur en méthane est supérieure à 25%, de préférence comprise entre 30% et 40%, de préférence encore de l'ordre de 35% et un perméat appauvri en méthane,
• un moyen d'alimentation des brûleurs de ladite chaudière à gaz avec le rétentat du second module de séparation,
un moyen d'évacuation du perméat issu du second module de séparation.

5. Installation selon la revendication 4 dans laquelle les moyens aptes à coopérer pour chauffer la matière organique contenue dans le digesteur comprennent des moyens complémentaires aptes à récupérer de la chaleur sur le module de prépurification du biogaz et le compresseur de biogaz prépurifié ainsi que des moyens complémentaires aptes à fournir la chaleur récupérée à l'eau du circuit de chauffage du digesteur.

6. Installation selon la revendication 4 ou la revendication 5 comprenant en outre des moyens aptes à recycler une fraction du rétentat du second étage de membranes à l'entrée du compresseur de biogaz prépurifié, les dits moyens comprenant une canalisation équipée d'une vanne de régulation de débit.

## Patentansprüche

1. Verfahren zur Herstellung von Biomethan, das ausgelegt ist, um ein Erdgasnetz zu versorgen, das ein Verfahren zur Lieferung von Wärme zur Heizung des Schritts der Herstellung von Biogas einschließt, wobei:
• das Verfahren zur Herstellung von Biomethan mindestens umfasst:
• einen Schritt (a) des Herstellens von Biogas durch Methanisierung von organischem Material,
• einen Schritt (b) des Vorbehandelns des in Schritt (a) hergestellten Biogases,
• einen Schritt (c) des Komprimierens des vorbehandelten Biogases,
• einen Schritt (d) des Trennens durch Permeation des vorbehandelten und komprimierten Biogases, das aus Schritt (c) stammt, um ein gasförmiges Retentat mit einem Methangehalt von mehr als 89 %, vorzugsweise mehr als 96,5 %, und ein gasförmiges Retentat mit einem Methangehalt, der zwischen 10 und 25 % liegt, vorzugsweise im Bereich von 20 %, herzustellen,
• einen Schritt (e) des Bereitstellens des gasförmigen Retentats von Schritt (d) als Biomethan,
• das Verfahren zur Lieferung von Wärme an den Schritt (a) des Herstellens von Biogas mindestens umfasst:
• einen Schritt (f) des Zuleitens von Wärme an den Schritt (a) über einen Heizwasserkreislauf,
• einen Schritt (g) des Heizens des Heizwassers in einem Gaskessel,
**dadurch gekennzeichnet, dass** das Verfahren zur Lieferung von Wärme umfasst:
• einen Schritt (h) des Trennens durch Permeation des gasförmigen Permeats, das aus dem ersten Schritt (d) des Trennens durch Permeation stammt, um ein gasförmiges Retentat, angereichert mit Methan, herzustellen, dessen Methangehalt größer oder gleich 25 % ist, vorzugsweise zwischen 30 % und 40 % liegt, noch bevorzugter im Bereich von 35 %, und ein methanarmes Permeat zu erzeugen,
• einen Schritt (i) des Versorgens der Brenner des Kessels von Schritt (g) mit dem Retentat des Schritts des Trennens (h),
• einen Schritt (j) des Entfernens des Permeats, hergestellt durch den Schritt (h) des Trennens durch Permeation.

2. Verfahren nach Anspruch 1, wobei das Verfahren zur Heizung von Schritt (a) einen Schritt (k) des komplementären Zuleitens von Wärme an das Wasser umfasst, das in dem Heizewasserkreislauf zirkuliert, ausgehend von der Wärme, die in den Schritten (b) und (c) wiedergewonnen wird.

3. Verfahren nach Anspruch 1 oder Anspruch 2, wobei eine variable Fraktion des Retentats, angereichert mit Methan, von Schritt (h) des Trennens an den Eingang des Schritts des Komprimierens (c) zurückgeschickt wird, wenn das Angebot an Wärme höher als die Anforderungen von Schritt (a) des Herstellens von Biogas ist.

4. Anlage zur Durchführung des Verfahrens nach Anspruch 1 zur Herstellung von Biomethan, die dazu bestimmt ist, ein Erdgasnetz zu versorgen, und der integrierten Lieferung von Wärme zur Heizung des Schritts der Herstellung von Biogas, umfassend mindestens Folgendes:
• eine Einheit zur Herstellung von Biomethan, umfassend mindestens:
• eine Quelle von organischem Material,
• einen Faulbehälter zur Herstellung von Biogas durch Methanisierung des organischen Materials,
• ein Modul zur Vorreinigung des erzeugten Biogases,
• einen Kompressor, der ausgelegt ist, um das vorgereinigte Biogas zu komprimieren,
• ein erstes Modul zur Trennung durch Membranpermeation des vorgereinigten und komprimierten Biogases, das ausgelegt ist, um ein gasförmiges Retentat mit einem Methangehalt von mehr als 89 %, vorzugsweise mehr als 96,5 %, und ein gasförmiges Permeat mit einem Methangehalt, der zwischen 10 und 25 % liegt, vorzugsweise im Bereich von 20 %, herzustellen,
• ein Mittel zum Bereitstellen des gasförmigen Retentats von Schritt (d) als Biomethan,
• Mittel, die ausgelegt sind, um zusammenzuwirken, um das organische Material zu heizen, das im Faulbehälter enthalten ist, umfassend mindestens:
• einen Heizwasserkreislauf, der ausgelegt ist, um den Faulbehälter zu heizen,
• einen Gaskessel, der ausgelegt ist, um das Wasser des Heizwasserkreislaufs zu heizen,
**dadurch gekennzeichnet, dass** die Mittel, die ausgelegt sind, um zusammenzuwirken, um das organische Material zu heizen, umfassen:
• ein zweites Modul zur Trennung durch Membranpermeation des gasförmigen Permeats, das aus dem ersten Modul zur Trennung durch Permeation stammt, das ausgelegt ist, um ein gasförmiges Retentat, angereichert mit Methan, dessen Methangehalt größer als 25 % ist, vorzugsweise zwischen 30 % und 40 % liegt, noch bevorzugter im Bereich von 35 %, und ein methanarmes Permeat herzustellen,
• ein Mittel zur Versorgung der Brenner des Gaskessels mit dem Retentat des zweiten Moduls zur Trennung,
ein Mittel zur Entfernung des Permeats, das aus dem zweiten Modul zur Trennung stammt.

5. Anlage nach Anspruch 4, wobei die Mittel, die ausgelegt sind, um zusammenzuwirken, um das organische Material zu heizen, das im Faulbehälter enthalten ist, komplementäre Mittel umfassen, die ausgelegt sind, um Wärme auf dem Modul zur Vorreinigung des Biogases und dem Kompressor von vorgereinigtem Biogas wiederzugewinnen, ebenso wie komplementäre Mittel, die ausgelegt sind, um die wiedergewonnene Wärme an das Wasser des Heizkreislaufs des Faulbehälters zu liefern.

6. Anlage nach Anspruch 4 oder Anspruch 5, weiter Mittel umfassend, die ausgelegt sind, um eine Fraktion des Retentats der zweiten Stufe von Membranen am Eingang des Kompressors von gereinigtem Biogas zu recyceln, wobei die Mittel eine Leitung umfassen, die mit einem Ventil zur Regulierung des Durchsatzes ausgestattet ist.

## Claims

1. Method for producing biomethane adapted to supply a natural gas network comprising a heat supply method for heating purposes during the biogas production step, wherein:
• the method for producing biomethane comprises at least:
• a step (a) whereby biogas is produced by methanisation of organic matter,
• a step (b) whereby the biogas produced in step (a) is pre-processed,
• a step (c) whereby the pre-processed biogas is compressed,
• a step (d) whereby the pre-processed and compressed biogas of step (c) is separated by permeation to produce a gaseous retentate featuring a methane content greater than 89 %, preferably greater than 96.5 %, and a gaseous permeate featuring a methane content ranging from 10 to 25 %, preferably of around 20 %,
• a step (e) whereby the gaseous retentate of step (d) is made available as biomethane,
• the heat supply step of step (a) for the production of biogas comprises at least:
• a step (f) whereby heat is supplied to step (a) through a heating water circuit,
• a step (g) whereby said heating water is heated in a gas-fired boiler,
**characterised in that** the heat supply method comprises:
• a step (h) whereby the gaseous permeate from the first step (d) of separation by permeation is separated by permeation to produce a methane-enriched gaseous retentate featuring a methane content of 25 % or more, preferably ranging from 30 % to 40 %, preferably still of around 35 %, and a methane-depleted permeate,
• a step (i) whereby the burners of the gas-fired boiler of step (g) are supplied with the retentate produced in the separation step (h),
• a step (j) whereby the permeate produced by the separation by permeation of step (h) is evacuated.

2. Method according to claim 1 wherein the heating method of step (a) comprises a step (k) whereby additional heat is supplied to the water circulating in said heating water circuit from the heat retrieved in steps (b) and (c).

3. Method according to claim 1 or claim 2 wherein a variable fraction of the methane-enriched retentate produced in the separation step (h) is returned to the input of the compression step (c) when the heat supply exceeds the demand of the biogas production step (a).

4. Installation to implement the method according to claim 1 for the production of biomethane used to supply a natural gas network and the integrated heat supply for heating purposes of the biogas production step comprising at least:
• a biomethane production unit comprising at least:
• a source of organic matter,
• a digester for the production of biogas by methanisation of said organic matter,
• a module for the pre-purification of the produced biogas,
• a compressor adapted to compress the pre-purified biogas,
• a first module for the membrane permeation separation of the pre-purified and compressed biogas adapted to produce a gaseous retentate featuring a methane content greater than 89 %, preferably greater than 96.5 %, and a gaseous permeate featuring a methane content ranging from 10 to 25 %, preferably of around 20 %,
• the means to supply the gaseous retentate of step (d) as biomethane,
• the means to cooperate for the purpose of heating the organic matter contained in the digester comprising at least:
• a heating water circuit adapted to heat the digester,
• a gas-fired boiler adapted to heat the water of said heating water circuit, **characterised in that** the means adapted to cooperate for the purpose of heating said organic matter comprises:
• a second module for the membrane permeation separation of the gaseous permeate produced in the first permeation separation module, adapted to produce a methane-enriched gaseous retentate featuring a methane content greater than 25 %, preferably between 30 % and 40 %, preferably still of about 35 %, and a methane-depleted permeate,
• the means to supply the burners of said gas-fired boiler with the retentate from the second separation module,
• the means to evacuate the permeate from the second separation module.

5. Installation according to claim 4 wherein the means adapted to cooperate for the purpose of heating the organic matter contained in the digester comprises additional means adapted to retrieve the heat from the biogas pre-purification and biogas compressor module, as well as additional means adapted to supply the retrieved heat to the water of the heating circuit of the digester.

6. Installation according to claim 4 or to claim 5 further comprising means adapted to recycle a fraction of the retentate from the second membrane step towards the input of the pre-purified biogas compressor, said means comprising a duct fitted with a flow regulation valve.
